# EUROPEAN PATENT SPECIFICATION

(11) **EP 1 461 086 B1**
(45) Date of publication and mention of the grant of the patent: **17.02.2010**
(21) Application number: 02804948.4
(22) Date of filing: 27.11.2002
(51) Int. Cl.: A61K 47/48

(54) **ENZYMATIC CLEAVABLE REAGENTS FOR SPECIFIC DELIVERY TO DISEASE SITES**
ENZYMATISCHE SPALTBARE REAGENZIEN ZUM SPEZIFISCHEN TARGETING VON KRANKHEITSHERDEN
REACTIFS CLIVABLES ENZYMATIQUEMENT PERMETTANT LE RELACHEMENT SPECIFIQUE DANS DES SITES PATHOLOGIQUES

(30) Priority: 17.12.2001 GB 0130104; 13.06.2002 GB 0213619
(43) Date of publication of application: 29.09.2004
(73) Proprietor: University College Cardiff Consultants Ltd., Cardiff, Wales CF24 0DE (GB)
(72) Inventor: MORGAN, B. P., Univ. of Wales College of Medicine, Cardiff CF14 4XN (GB); HARRIS, C. L., Univ. of Wales College of Medicine, Cardiff CF14 4XN (GB)
(74) Representative: Fyles, Julie Marie
(86) International application number: PCT/GB2002/005371
(87) International publication number: WO 2003/051393

(56) References cited:
- EP-A- 0 712 635
- WO-A-01/68145
- WO-A-98/19705
- MAKRIDES SAVVAS C: "Therapeutic inhibition of the complement system." PHARMACOLOGICAL REVIEWS, vol. 50, no. 1, March 1998 (1998-03), pages 59-87, XP002247613 ISSN: 0031-6997
- MERCURI FRANCESCA A ET AL: "Recombinant human aggrecan G1-G2 exhibits native binding properties and substrate specificity for matrix metalloproteinases and aggrecanase." JOURNAL OF BIOLOGICAL CHEMISTRY, vol. 274, no. 45, 5 November 1999 (1999-11-05), pages 32387-32395, XP002247614 ISSN: 0021-9258
- HARRIS CLAIRE L ET AL: "Complement regulator-Fc fusion proteins as prodrugs for efficient delivery of active regulators to sites of disease." INTERNATIONAL IMMUNOPHARMACOLOGY, vol. 2, no. 9, August 2002 (2002-08), page 1316 XP009013764 XIX International Complement Workshop;Palermo, Italy; September 22-26, 2002, August, 2002 ISSN: 1567-5769

## Description

### Field of the Invention

This invention relates to therapeutic reagents that can be manipulated to have a reduced effect or activity when circulating in the body of a host, but which are designed to be released in an active form at specific sites and times when needed. In particular, the invention relates to regulators of immune functions such as anti-complement reagents, which may be used to regulate the negative roles of immune molecules or cells in the host. Further, the invention relates to the use of said therapeutic reagents, pharmaceutical compositions including same and methods of medical treatment.

### Background of the invention

The complement (C) system is an important component of the immune system of hosts, including humans and animals. C is known to consist of a number of proteins that act in a proteolytic cascade to target antigens or cells. During this sequence, one protein activated through binding antigen cleaves the next reacting protein to generate a new activated proteolytic enzyme, which cleaves and thereby activates the next protein in the sequence. Proteolytic fragments and protein complexes generated during activation have phlogistic activity, and cause inflammatory tissue changes such as increased vascular permeability and attraction of polymorphonuclear leukocytes.

Examples of such proteins include C5a, C3a and MAC (cytolytic macromolecular membrane attack complex). Targeting of a cell by C results in cell damage or death directly through formation of MAC, or indirectly through initiation of inflammation due to production of the inflammatory mediators (C5a, C3a and MAC) and phagocytosis of C targeted cells.

To protect themselves from this potentially harmful defence mechanism, cells express, on their surface, complement regulatory proteins (CRegs) which rapidly and efficiently inactivate 'accidental' foci of C activation (Morgan, B & Harris C., Complement Regulatory Proteins (1999)).

CRegs function either by inactivating enzymes, such as the C3 and C5 convertases, which are formed during C activation and which are responsible for cleavage of C3 and C5, or by interfering with MAC formation. In humans, the C regulators membrane cofactor protein (MCP;CD46), decay accelerating factor (DAF;CD55) and complement receptor 1 (CR1;CD35) inhibit C by accelerating the decay of or (with factor I) irreversibly inactivating the C3 and C5 convertase enzymes. A fourth regulator, CD59, inhibits MAC formation by binding C8 and/or C9, and inhibiting C9 polymerisation during MAC formation.

In normal circumstances, these control mechanisms are sufficient to protect cells from damage by homologous C. However, evidence of C activation is abundant in diverse inflammatory diseases including rheumatoid arthritis (RA), systemic lupus erythematosus (SLE), glomerulonephritis, adult respiratory distress syndrome (ARDS), ischemia-reperfusion injury, demyelination, myaesthenia gravis, Arthus reaction, rejection in transplantion, lupus nephritis and multiple sclerosis. For example, in RA, soluble products of C activation are abundant in the synovial fluid of affected joints. Complement deposits are evident in synovial tissue, together with leukocytes (neutrophils and T cells) attracted to the site by a gradient of C5a and other chemo-attractants.

Whilst C itself is not always the primary cause of disease, it acts to sustain the pro-inflammatory cycle, can exacerbate the disease and perpetuate and extend tissue damage due to non-targeted activity.

### Description of the Prior Art

Research has been carried out to find therapeutic reagents capable of inhibiting the C cascade and preventing the formation of pro-inflammatory mediators. Some groups have concentrated on high-throughput screening to identify small chemical compounds that might inhibit C. Others have exploited the naturally occurring cell-associated CRegs by generation of soluble, recombinant forms of these molecules that inhibit C through their native activities. Yet others have developed antibodies that block activation of specific components in the C system.

Anti-C reagents are known, most of which inhibit production of C5a and MAC, the key active by-products of C activation. The best-described examples are, first, a scFv that binds C5 and prevents its enzymatic cleavage, and second, a soluble, recombinant form of CR1 (sCR1) that inhibits the amplification enzymes in the activation pathways of C. Both of these reagents have been used in acute conditions, such as adult respiratory distress syndrome (ARDS), or ischaemia-reperfusion injury, which occurs in many clinical contexts, including myocardial infarction following cardiopulmonary bypass.

Known reagents, with the exception of sCR1, have low molecular weights, for example, from 12 kDa for CD59 to 40-50 kDa for DAF, MCP and Crry, therefore, these reagents are rapidly cleared from the body *via* the kidneys.

In addition to development of sCR1 for anti-C therapy, soluble recombinant forms of other human and rodent C regulators have been generated and tested in C-mediated inflammatory conditions, such as the Arthus reaction and rejection in xenotransplantation. In most combinations, human C regulators also inhibit rodent C and rodent C regulators control human C. For example, it has been shown that human and rodent DAFs are not species-specific in their complement inhibiting activities (Harris et al Immunology 100 462-470 (2000)). However, administration of a foreign C regulator results in a prompt immune response in the recipient, limiting its function to just a few days. This has restricted the ability to test human C regulators, such as sCR1, in chronic disease models in rodents.

Various modified forms of the C regulators have been produced and tested. Attempts have been made to combine two regulatory activities into one reagent, but these attempts have resulted in linear, inflexible molecules where the CRegs are fused end-to-end, making them unsuitable for targeted action (Higgins et al in J Immunol 158 2872 (1997)). A chimeric molecule, in which mouse Crry has been fused to mouse IgG1 domains has been produced (Quigg et al J Immunol 4553 (1998)). This has been used in the therapy of murine glomerulonephritis.

Known reagents, including sCR1, have short half-lives *in vivo* (minutes to hours), requiring frequent systemic administration and limiting their roles to the therapy of acute situations. They are not suitable for long-term use in the treatment of chronic disease.

In the aforementioned paper by Harris *et al* (2000), human DAF-Ig has been produced, in which DAF is tethered to the Fc fragment of an immunoglobulin molecule. Tests *in vitro* and *in vivo* have demonstrated that Ig fusion proteins, such as DAF-Igs, have anti-C activity. Subsequent *in vivo* tests have demonstrated the ability of fusion proteins to remain in the circulation and to inhibit plasma C activity over longer periods of time.

A confounding problem with current CReg-based anti-C reagents is that C activity is inhibited systemically. Although this may be of little consequence in acute situations, long-term reduction in systemic C activity is not desirable. This is because long-term inhibition of C may predispose individuals to infection, and also severely compromise the C-dependent process of immune complex solubilization and clearance.

We set out to overcome the problems of known reagents, specifically short half-life in *vivo* and systemic inhibition of C, by engineering a therapeutic reagent that is long-lived in the circulation and has little or no C inhibitory activity while in the circulation, but which can be activated at sites of inflammation and/or C activation. Such an anti-C "pro-drug" would offer advantages over current reagents, which treat acute situations of C activation, and allow for the first time use of an anti-C therapeutic reagent in the treatment of chronic illnesses in which C activation is implicated.

We have taken advantage of our observation that, in some CReg-Ig fusion proteins, the C regulator has much reduced or absent C regulatory function. Release of the CReg from the Ig moiety restores C regulatory capacity. Inclusion of one or more specific enzyme cleavage sites between the CReg and the hinge region of the Ig moiety can provide a therapeutic reagent that can possess the desirable properties of a long half-life *in vivo,* minimal systemic disturbance and efficient C regulation at the site of inflammation or disease.

### Summary of the Invention

Accordingly, the present invention seeks to provide therapeutic reagents having a long plasma half-life, minimal systemic effects and an efficient function at the site of pathology. Delivery to the appropriate site may be enhanced by the inclusion of targeting elements. These reagents include anti-C agents. These therapeutic agents differ from previously-described reagents, in that the intact therapeutic reagent or 'prodrug' is designed to express little or no systemic activity. Instead, sites are engineered between the active agent, which is a regulatory moiety, and a carrier moiety, such as an Ig, whereby the agent is released in an active form at the site of pathology to mediate its therapeutic effect. For example, inhibition of the C cascade at sites of inflammation can be achieved using a prodrug comprising a CReg attached *via* a cleavable sequence to a Ig Fc domain. The Ig moiety is chosen both to minimise C regulatory function of the attached CReg in prodrug form and to maximise the half-life of the CReg-Ig prodrug in the circulation. The therapeutic reagents may therefore be viewed as prodrugs when circulating in the body and active drugs following release of the CReg or other active agent at the target site.

In addition, the therapeutic reagent preferably further comprises a specific targeting sequence that can enhance delivery to the site of disease.

Described herein is a therapeutic reagent to control one or more reactions of the immune system in a host, said therapeutic reagent comprising:
i. at least one regulatory moiety that is an immunoregulatory protein (IRP) or a functional fragment thereof;
ii. a carrier protein which renders said IRP inactive or substantially inactive; and
iii. positioned therebetween at least one cleavage site;
whereby
when the regulatory moiety is at, or adjacent, target organ or tissue in the host, said cleavage site is cleaved, freeing the regulatory moiety from the carrier protein and restoring its regulatory activity.

Described herein is also a therapeutic reagent that is inactive systemically comprising:
i. at least one regulatory moiety that has a therapeutic activity;
ii. a carrier protein which renders said regulatory moiety inactive or substantially inactive; and
iii. positioned therebetween at least one cleavage site characterised in that said cleavage site is a substrate for a matrix metalloproteinase (MMP) or an aggrecanase; whereby
at sites where MMP's or aggrecanase are active in the host said cleavage site is cleaved and said regulatory moiety is freed from the carrier protein and so able to perform its therapeutic function.

Described herein is also a therapeutic reagent to control one or more reactions of the immune system in a host, said therapeutic reagent comprising:
i. at least one regulatory moiety that is an immunoregulatory protein (IRP) or a functional fragment thereof:
ii. a carrier protein which renders said IRP inactive or substantially inactive; and
iii. positioned therebetween at least one cleavage site characterised in that said cleavage site comprises a substrate for at least one enzyme of the Complement system; whereby
when said therapeutic reagent is at or adjacent a site in the host where Complement is active said cleavage site is cleaved so freeing the immunoregulatory moiety from the carrier protein and enabling it to perform its immunoregulatory activity.

The term "regulatory moiety" is used to mean the part of the therapeutic reagent that acts or causes an effect in the host.

Preferably, the cleavage site is between the regulatory moiety and the carrier protein. More preferably, the cleavage site is positioned between the regulatory moiety/CReg and a hinge region of the carrier protein/Ig.

It is preferred that the cleavage site comprises an extrinsic moiety at which the therapeutic reagent can be cleaved under cleavage conditions. More preferably, it comprises an extrinsic amino acid or protein sequence, which is inserted between the regulatory moiety and the carrier protein. By extrinsic, is meant that the amino acid or protein sequence is not naturally a component of the regulatory moiety or the carrier protein and must therefore be inserted in the therapeutic reagent together with the regulatory moiety and carrier protein. Ideally, the cleavage site is susceptible to cleavage by enzymes of the matrix mettaloproteinase (MMP) and aggrecanase families. Secondarily, it is susceptible to enzymes of the complement system. It therefore follows that a therapeutic reagent employing these latter cleavage sites will become active at locations where complement is active because complement enzymes will cleave the cleavage site and so release the regulatory moiety which can then function in a regulatory manner to achieve its therapeutic effect.

Nevertheless, the cleavage site may comprise intrinsic amino acids or proteins that are already present, for example as part of the carrier protein or regulatory moiety. Again, the amino acids or proteins may be acted on by enzymes at a particular site so that the regulatory moiety and the carrier protein become cleaved, thereby enabling the therapeutic agent to act at a site in the host's body. However, in either case, it must be ensured that no cleavage site is present in the therapeutic reagent that would result in significant cleavage thereof to release the regulatory moiety in active form other than at or adjacent the target organ or tissue; which, in the instance where the cleavage site is susceptible to cleavage by complement enzymes would be at sites where complement was active.

The regulatory moiety is a C regulatory protein (CReg). Ideally, the CReg is an immunoregulatory protein that acts either as a decay accelerating factor or a cofactor for the plasma protease factor 1 or to inhibit formation of membrane attack complex. In one embodiment of the invention the regulatory moiety may also be a combination of CReg and other regulatory proteins. It is envisaged that the CReg may be any of those described (Morgan & Harris in Complement Regulatory Proteins (1999)), including those mentioned above, especially DAF, CD59, Crry, active fragments of CR1 and MCP, and may also include active fragments of factor H (FH) or other C regulators. Further, the regulatory moiety may comprise more than one active agent, such as more than one CReg. Alternatively, and more preferably, a therapeutic reagent comprising a single active agent may be co-admirustered with another therapeutic reagent containing a single, but different, regulatory moiety.

It is preferred that the carrier protein is an immunoglobulin (Ig) Fc fragment. Suitable Igs include IgGI, IgG2, IgG3 or IgG4, with IgG4 being a preferred Ig and IgG2 especially preferred. Modifications of the Ig to minimise activity of the prodrug-bound CReg, to extend plasma half-life or to minimise effector functions of the Fc are included. In one embodiment of the invention the Fab arms of the Ig may be replaced by two CReg moieties. Ideally, the immunoglobulin is human immunoglobulin.

The therapeutic reagent may comprise an immunoglobulin in which one arm comprises a CReg, while the other may comprises a targeting moiety such as a Fab specific for a certain cell or tissue or an adhesion molecule specific for a certain cell or tissue. Targeted reagents may also include a Fab on one arm of the Ig while the other arm comprises a different regulatory moiety, such as a protein that modulates other types of immune response, eg an anti-cytokine agent or a cytokine receptor blocker.

The cleavage site of the therapeutic reagent is enzyme based.

The cleavage site may comprise a polypeptide/amino acid sequence in the prodrug susceptible to a specific enzymatic cleavage. The enzyme is that present at sites of inflammation or immuno pathology, for examples matrix metalloproteinases (MMPs) and/or aggrecanases. The cleavage site(s) in the therapeutic reagent can be cleaved by specific enzymes such as MMPs and aggrecanases at the target site to release the CReg, or other active agent acting as an immunoregulatory moiety, from the carrier protein, such as an Ig Fc domain, in order to restore function of the active agent.

In a preferred aspect, the therapeutic reagent comprises a cleavage site that itself comprises a polypeptide/amino acid sequence incorporated between the regulatory moiety/CReg and the hinge region of an Ig, the cleavage site being susceptible to cleavage by one or more enzymes, selected from: MMP3, MMP8 and other members of the MMP family, and those of the aggrecanase family. Examples of such are mentioned by Mercuri et al in J Biol Chem 274 32387 (1999). Several publications describe the preparation of recombinant aggrecanase, such as Tortorella et al in Science 284 1664 (1999) [aggrecanase-1] and Horber et al in Matrix Biol 19 533 (2000) Other enzymes expressed specifically or in increased abundance at the target site may also be utilised, with appropriate modification of the cleavage site.

In the Examples hereinbelow, a preferred cleavage sequence is a part of the inter-globular-domain (IGD) of aggrecan, which comprises approximately 120 amino acids. Ideally said cleavage sequence comprises the minimum number of amino acids needed for cleavage to occur. It is preferred that the cleavage sequence for aggrecanase or MMPs comprises in the range of from 17-75 amino acids. More particularly, the cleavage sequence may comprise the aggrecanase IGD cleavage site itself.

Preferred MMP or aggrecan cleavage amino acid sequences comprise:
IGD1 having the amino acid sequence RNITEGEARSVILTVK;
IGD2 having the amino acid sequence TTFKEEEGLGSVELSGL; and
IGD75 having the amino acid sequence: GYTGEDFVDIPENFFGVGG-EEDITVQTVTWPDMELPLPRNITEGEARGSVILTVKPIFEVSPSPLEPEEPFTFAP.

It is preferred that the therapeutic reagent is functionally substantially inactive prior to cleavage. By 'substantially inactive' is meant that the therapeutic reagent has no, or at least a much reduced ability to act on the host, compared to when the regulatory moiety is in its free and/or solubilised state. In this state, the therapeutic reagent can therefore be described as a prodrug. In the case of the therapeutic reagent comprising a CReg, the reagent has a significantly reduced ability to act on the host's C system compared to when the CReg is not bound to carrier. For example, at least an order of magnitude reduction in activity can be observed, such as in the range of from a 10- to 60-fold reduction. When comparing molecules on a 'moles of CReg' basis (ie taking into account the mass of the Ig domains), not by mass of reagent, then:
(1) Rat DAF-IgG1 has a 10-fold decrease in ability to regulate the classical pathway of complement, compared to soluble DAF (comprising four short consensus repeats [SCRs]);
(2) Rat CD59-IgG1 has a 35-fold decrease in ability to regulate the terminal pathway of complement, compared to free soluble CD59;
(3) Human DAF-IgG2 or DAF-IgG4 has a 10-fold decrease in activity (classical pathway), compared to soluble DAF (four SCRs); and
(4) Human DAF (3 amino terminal SCR)-IgG2 has a 60-fold decrease in ability to regulate classical pathway, compared to soluble DAF (three SCRs).

When cleavage occurs, the therapeutic reagent is activated, by release of the immunoregulatory moiety/CReg from the carrier protein, so that, in the case of the CReg, for example, it can participate in the control of C. The principle applies equally to other immune regulators delivered as Ig fusion proteins. By 'activated', we therefore mean that the activity of the CReg or other active agent is more or less equivalent to that of the agent in its non-prodrug-bound/soluble form.

For human DAF, in particular, it has been noted that the choice of antibody isotype greatly influences flexibility at the hinge region of the DAF-Ig fusion protein, which in turn influences the activity of the DAF *in vitro* and *in vivo.* Altering the activity of the regulatory moiety can be used to control its effect on the host when circulating in the body. Fusion to either IgG2 or IgG4 Fc domains has the most restrictive effect on function of DAF. This is likely to be due to steric hindrance around the hinge region. Similar principles will apply to the design of other prodrugs to provide reagents with markedly restricted function but that are activated upon removal of the Fc by cleavage.

It is envisaged that the cleavage site is positioned between the regulatory moiety/CReg and a joining or hinge region of the carrier protein/antibody.

Preferably, the therapeutic reagent includes the minimal portion of the CReg or other agent necessary for function upon release.

A further embodiment of the invention relates to a targetable therapeutic reagent comprising a regulatory moiety-carrier protein prodrug as described above, in which one of the Fab arms of the Ig is replaced by a CReg or other immune regulatory molecule and the other by a targeting moiety comprising either a Fab or another protein that confers specific binding in the target tissue.

Accordingly, the present invention further provides a therapeutic reagent to control one or more pathologies in a host, said reagent comprising a immunoregulatory moiety and a carrier protein, characterised in that there is a cleavage site between the immunoregulatory moiety and the carrier protein, whereby, when the immunoregulatory moiety is at or adjacent a target in the host, cleavage of the therapeutic reagent occurs at the cleavage site, freeing the regulatory moiety from the carrier protein, wherein the therapeutic reagent is combined with a tissue or cell-specific targeting moiety.

Preferably, the targeting moiety is one or more membrane targeting molecule(s). These enable the therapeutic reagent to be localised to cell membranes. For example, a targeting moiety may comprise an addressin (described below) which is incorporated into the therapeutic reagent between the regulatory moiety and the cleavage site such that, following cleavage, the regulatory moiety and the addressin are released in a bound form, and the addressin is thus able to direct, or target, the regulatory moiety to a cell membrane.

For example, in the case of CReg-Ig fusion proteins, incorporation of a membrane-targeting molecule can yield therapeutic reagents that have minimal systemic anti-C activity, and that can bind to membranes but only become active when released at sites of expression of the relevant enzymes. Membrane targets might include adhesion molecules, or C fragments deposited in and around inflamed tissue.

As mentioned, membrane targeting may involve the engineering of a myristate group together with a stretch of negatively charged amino acids into the protein, termed an 'addressin' (Smith & Smith in Mol Immunol 38 249-55 (2001)). Together, these modifications confer upon the protein the propensity to associate with lipid membranes through insertion of the myristate and charge interactions of the amino acids with negatively charged phospholipid headgroups. Just one example of a CReg modified with an addressin is APT070, which comprises the three amino-terminal SCR of CR1 attached to an addressin at the carboxy terminus. Anti-C prodrugs modified in this way will bind lipid membranes and subsequent enzymatic cleavage will release active C regulator at tissue site, or visa versa. Additional targeting strategies may include the sLe^{x} carbohydrate moiety, a ligand for E- and P-selectins on activated endothelia.

Yet a further embodiment of the invention relates to DNA coding for a therapeutic reagent as described above. In particular, the invention provides a method for preparing such a therapeutic reagent, which method comprises ligating DNA molecules each encoding the regulatory moiety, the carrier protein and the cleavage site comprising the therapeutic reagent and giving expression to the DNA sequence therby encoding the therapeutic reagent.

Preferably, the therapeutic reagent protein is expressed in a eukaryotic system using a high expression vector. A preferred expression vector is pDR2ΔEF1α (as described by Charreau in Transplantation 58 1222 (1994)), although other vectors may also be used.

A further embodiment of the invention relates to a culture system comprising the cDNA encoding the therapeutic reagent protein as described above inserted in a high expression vector and transfected in CHO cells or other appropriate eukaryotic expression systems, including DNA encoding a regulatory moiety and a carrier protein, separated by DNA encoding a cleavage site.

According to a further aspect of the invention there is provided the use of a therapeutic reagent, as aforedescribed, in the preparation of a medicament for the treatment of disease.

It is preferred that the therapeutic reagent is suitable for treating humans.

Diseases which may be treated include all those in which complement plays a role in pathology. Such diseases include inflammatory diseases, such as rheumatoid arthritis; immunological disorders *eg*. Arthus reaction; ischaemic disorders or cancer. Further conditions that may be treated include adult respiratory distress syndrome (ARDS), systemic lupus erythematosis, multiple sclerosis and other demyelinating disorders, glomerulonephritis, ischemia-reperfusion injuries, such as stroke and myocardial infarction, myaesthenia gravis, allergic reactions such as asthma and dermatological disorders, and rejection in transplantation.

It is envisaged that the therapeutic reagent may be administered systemically, such as *via* the intravenous, intramuscular or subcutaneous routes. Intravenous administration is particularly applicable where multiple sites in the body are involved, as, for example, in autoimmune disease. In some circumstances, the agent may be injected directly to a site of inflammation, such as intra-articularly in an inflamed joint in arthritis.

According to a further aspect of the invention there is provided a pharmaceutical composition including the therapeutic reagent, as aforedescribed, which is combined with a pharmaceutically acceptable carrier, which carrier comprises those conventionally known in the art.

The reagents could be used for a range of diseases for both human and veterinary applications.

The invention will now be illustrated by the following Examples, in which reference is made to the accompanying Figures.

### Brief Description of the Figures

**Figure 1** shows the results of new studies of in vivo half-life of DAF-Ig and soluble DAF (sDAF). Radiolabelled DAF-Ig (•) or sDAF (o) was administered to rats, blood was removed at certain timepoints and protein bound radioactivity was determined. Results are expressed as percent of levels at 3 minutes and represent the means of five animals ±SD.
**Figure 2** illustrates the therapeutic effect of DAF-Ig on antigen induced arthritis, a rat model of rheumatoid arthritis. Methylated BSA was introduced into the right knee of immune rats. DAF-Ig (○) or saline control (•) was administered to the joint at the same time. Swelling of the joint was measured daily and compared to that of the left knee. Results represent the mean of five animals ±SD. These results show a reduction in swelling and disease severity in treated compared with control animals from day 2 onwards. *p < 0.01, **p < 0.001
**Figure 3** shows new studies concerning in vitro complement regulatory function of DAF-Ig and the effect of cleavage by the enzyme, papain, that cleaves the DAF-Ig and releases active DAF. a) Antibody sensitised erythrocytes were incubated in GVB with rat serum and different concentrations of sCR1 (□ ), DAF-Ig (Δ), sDAF (∇) or a non-regulatory Ig fusion protein ( O). b) shows results after treatment with papain. Haemolysis was assessed by release of haemoglobin to the supernatant and percent lysis was determined. Results represent the mean value ±SD of three determinations.
Figure 4a) shows the results of new studies concerning the in vitro complement regulatory function of CD59-Ig and the effect of using a spacer in CD59 fusion proteins. Guinea pig erythrocytes bearing C5b-7 sites were incubated in PBS/EDTA with rat serum and different concentrations of test protein. Results represent the mean value ±SD of three determinations showing the functional comparison of CD59-Ig (□), CD59-spacer-Ig (0), a non-regulatory Ig fusion protein (O) and sCD59 (•). b) shows results after treatment with papain. As seen, cleaved CD59 activity is comparable with sCD59 activity.
**Figure 5** shows an example of the results of a haemolytic assay showing the ability of different human DAF-Ig fusion proteins (DAF- G2, DAF-G4, S3- G4, S3 -G2) to inhibit complement, compared with inhibition achieved by sCR1 and soluble DAF with no Fc attached. DAF-G2, DAF-G4, four SCR of DAF attached to Fc of IgG2 and IgG4 respectively; S3-G2, S3-G4, three SCR of DAF attached to Fc of IgG2 and IgG4 respectively; SCRs1-3, SCRs1-4, soluble DAF with three and four SCR domains respectively and no Fc.
**Figure 6** shows a schematic representation of a therapeutic reagent according to the invention having CReg and IgFc moieties, together with a cleavage site.
**Figure 7** shows the portions of IGD of aggrecan (from 17-75 amino acids) incorporated into DAF-Ig of the invention, between the antibody hinge and DAF. Single underlined: major MMP cleavage site (including MMP3 and MMP8). Double underlined: major aggrecanase cleavage site, also cleaved by MMP8. Dotted underlined:alternative aggrecanase cleavage site.
**Figure 8** DNA sequences encoding different lengths of the IGD of aggrecan were cloned into the expression vector between human DAF and human IgG4 hinge. Lane (1) no IGD, (2) IGD 1 (3) IGD 2 (4) a control 'scrambled' polypeptide sequence (5) 75 amino acids of IGD. Supernatent from expressing cells were subject to SDS-PAGE and Western blot. Blots were probed with (a) anti-human Fc or (b) anti-human DAF.
**Figure 9** Human DAF-Ig containing 75 amino acids of IGD was purified by protein A affinity chromatography and subjected to SDS-PAGE. The lower band represents DAF-Ig which is not disulphide bonded at the hinge, this is characteristic of human IgG4. Gel filtration studies indicate that the 'half-forms' are linked through non-covalent bonds under non-denaturing conditions.
**Figure 10** The prodrug shown in figure 9 (1.5 µg) was digested with MMP3 or MMP8 and subjected to non-reducing SDS PAGE and Western blot. Blots were probed with polyclonal anti-human DAF. Lane 1: no enzyme; Lanes 2-4: 414, 138 and 46ng cdMMP3, respectively; Lanes 5-7; 300, 100 and 37ng MMP8 respectively.
**Figure 11** shows a schematic of the cleavage sites on a therapeutic reagent following cleavage of the prodrug described in the invention, detected by anti-neoepitope antibodies. BC3, new N-terminus at site 1; BC4, new C-terminus at site 3; BC14, new N-terminus at site 3.
**Figure 12** shows a prodrug as shown in Figure 6, digested with MMP8 or aggrecanase and subjected to reducing SDS PAGE and Western blot. Anti-neoepitope antibodies were used to detect the new N-termini following cleavage as described above.
**Figure 13** shows detection of the new C-terminus of a prodrug as shown in Figure 6, following cleavage at the major MMP site.
**Figure 14** shows cleavage ofDAF (4 SCRs)-IGD75-IgG4 with MMP3 and MMP8 using silver stained SDS PAGE gels.
**Figure 15** shows cleavage ofDAF (4 SCRs)-IGD1-IgG4 with MMP8 using silver stained SDS PAGE gels.
**Figure 16** schematically shows human S3-DAF-Ig2 (three SCR of DAF attached to IgG2 Fc) incorporating a DIPEN cleavage site.
**Figure 17** graphically shows the results of functional tests using the prodrug of Figure 16.
**Figures 18 to 22** show results of tests on the prodrug of Figure 16 as follows.

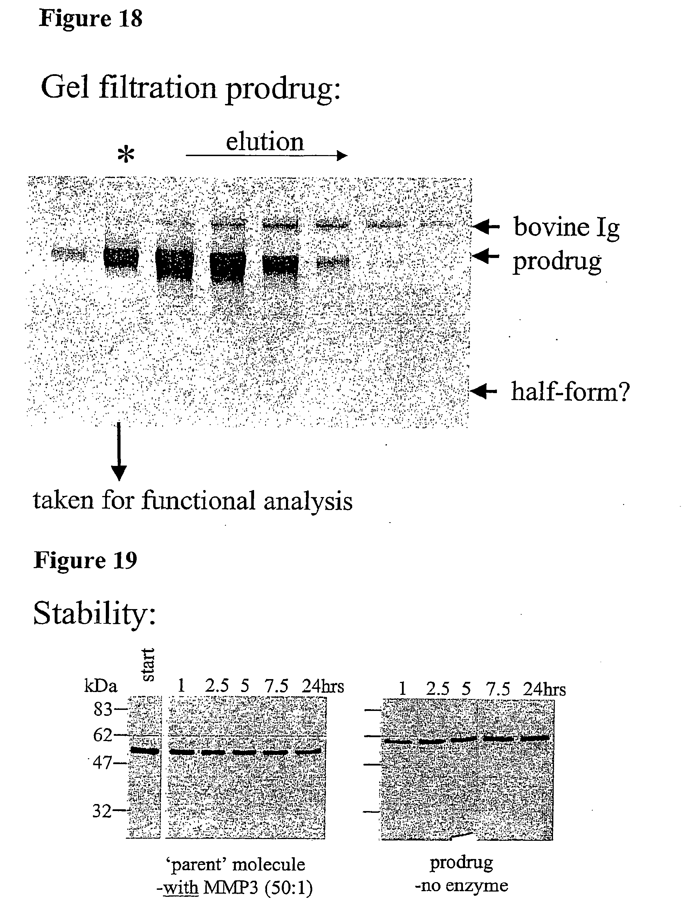

Figure 18: gel filtration to purify.
Figure 19: resistance of parent molecule to MMP3 cleavage at 37°C and stability of prodrug (no degradation) at 37°C.

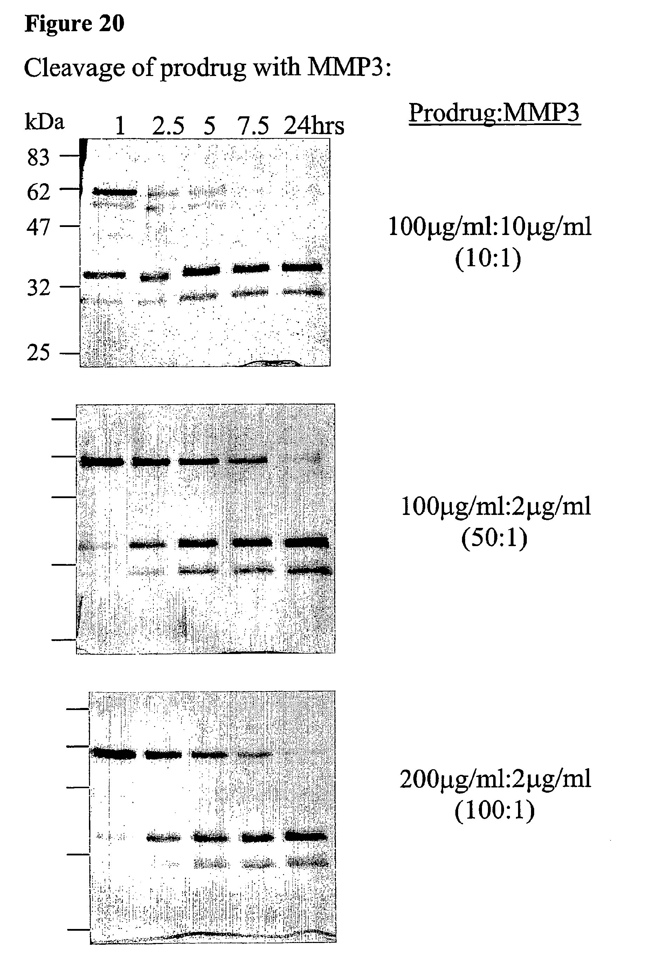

Figure 20: cleavage of prodrug by incubation with MMP3 at various doses at 37oC for up to 24 hours.

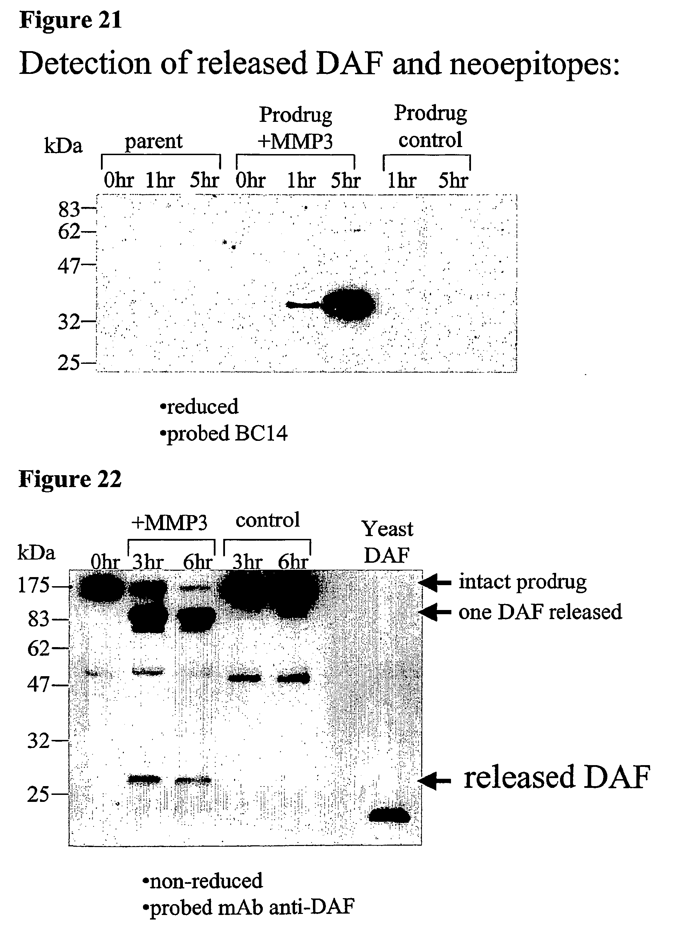

Figure 21: detection of neoepitopes following cleavage by MMP3.
Figure 22: detection with anti-DAF mAb of release of DAF from prodrug.

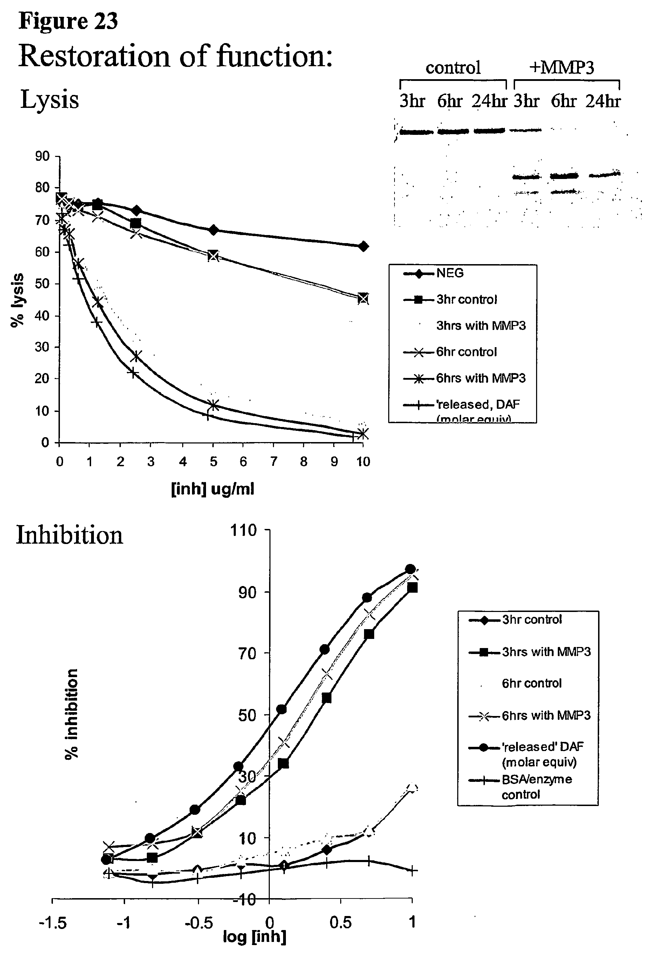

**Figure 23** graphically shows restoration of function and inhibitory activity of the prodrug versus controls. Controls: comprised the prodrug without MMP3. Inset: gel to illustrate cleavage of prodrug by MMP3 at 3, 6 and 24 hours.

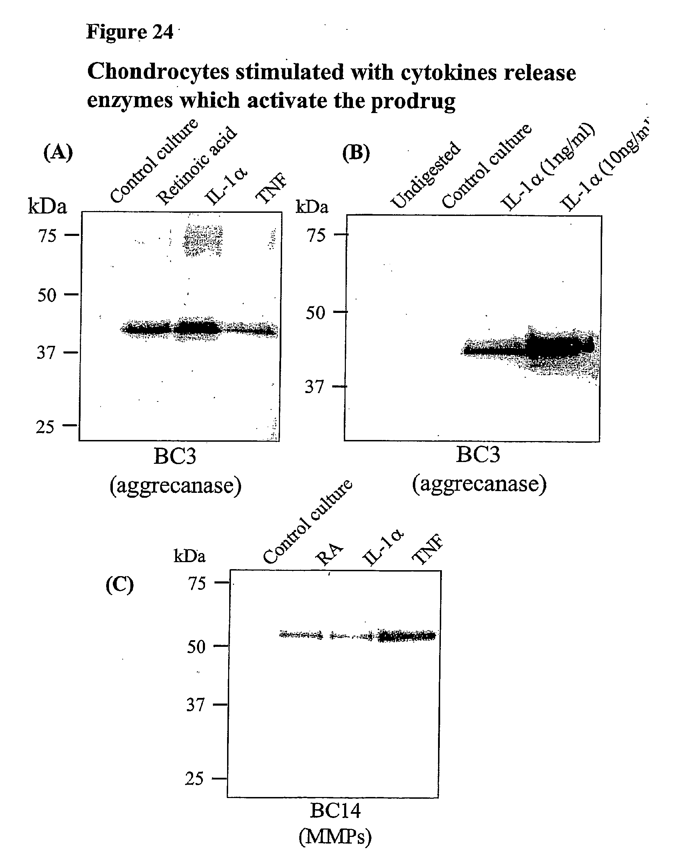

**Figure 24** comprises three sets of results (A B and C) on the prodrug comprising 4 SCR of DAF, 75 amino acids of IgD and IgG4 Fc, to determine whether enzyme released from chondrocytes treated with inflammatory cytokines, rather than purified enzyme, could cleave the prodrug. As can be seen, in each instance, the enzymes are effective and the prodrug is activated.

### EXAMPLES

### Preparation of recombinant proteins

### Method Example 1: - Rat DAF-Ig and CD59-Ig

DNA encoding the four SCRs of rat DAF was cloned into the expression vector SigpIg (R&D Systems) and that encoding the signal peptide and entire extracellular domain of CD59, omitting the GPI anchor signal sequence, was cloned into the vector pIgPlus (R&D Systems). In both cases DNA encoding the regulator was cloned upstream of and in frame with DNA encoding the hinge and Fc Domains of human IgG1. In order to achieve high levels of expression, DNA encoding the signal peptide, regulator and Ig domains was then sub-cloned using PCR into the high expression vector pDR2 Δ EF1α. CHO cells were transfected using lipofectamine (Life Technologies) according to the manufacturer's instructions and stable lines were established by selection with 400 µg/ml Hygromycin B (Life Technologies). Supernatant was collected and passed over a Prosep A column (Bioprocessing Ltd, Consett, UK) to purify the fusion protein. The column was washed with PBS and with 0.1M citrate buffer pH5.0 to remove contaminating bovine Ig and the fusion protein was eluted with 0.1M Glycine/HCl pH2.5. Eluted protein was neutralised with Tris, concentrated by ultrafiltration and dialysed into PBS.

### Method Example 2:- Soluble Rat DAF and soluble CD59

DNA encoding the signal peptide and four SCRs of rat DAF (C-terminal residue Lys254) was cloned directly into the expression vector pDR2 Δ EF1α. CHO cells were transfected as described above. sDAF was prepared by affinity chromatography on a monoclonal anti-DAF (RDII-24) column. Protein was eluted using 50mM diethylamine pH11 and immediately lyophilised. The dried protein was solubilised in phosphate buffer with 1M NaCl and was applied to a Superose 12 gel filtration column (Amersham-Pharmacia Biotech AB, Uppsala, Sweden). Proteins were eluted with PBS and fractions containing DAF were identified. The pure DAF was concentrated by ultrafiltration. Soluble CD59 containing the entire extracellular portion (omitting the GPI anchor) was also produced in transfected CHO cells and was obtained from idENTIGEN^{cyf} (Cardiff, UK).

### Method Example 3:- Control SCR Fusion Protein

A control SCR-containing fusion protein was also prepared in an identical manner to that of Example 1. This protein had no C-regulatory function.

### Method Example 4: CD59-Spacer-Ig according to the Invention

A CD59-containing fusion protein was also prepared in which the amino acids (Ser-Gly-Gly-Gly-Gly)₂-Ser were inserted between CD59 and the antibody hinge using two stage PCR. Briefly, DNA encoding CD59 and the Ig domains was reamplified in two separate reactions using new primers that incorporated the sequence of the spacer domain at the 3' end of CD59 and at the 5' end of the Ig hinge. The two PCR products were mixed together and allowed to anneal at complementary DNA sequences encoding the spacer domain. Following PCR using outside primers, the product was ligated into the expression vector pDR2 Δ EF1α. Cells were transfected and the second CD59-Ig protein was purified as described above. Protein concentrations were determined using Pierce Comassie assay (Perbio Science UK Ltd, Tattenhall, UK) using bovine serum albumin as a standard.

CD59-Ig has a mass of 77Kda, CD59-spacer-Ig has a mass of 78.5Kda and DAF-Ig has a mass of 122Kda, these masses being confirmed by mass spectrometry.

### Method Example 5:- Human DAF-IgG2 and Human DAF-IgG4 according to the invention

Human DAF-IgG1 was generated as described by Harris CL et al. (2000), Immunology, 100, 462. Fusion proteins consisting of human DAF and either IgG2 or IgG4 hinge were generated as follows:

DNA encoding the hinge and Fc of human IgG4 or IgG2 were amplified by RT-PCR from human peripheral blood lymphocyte RNA. The amino terminal sequences of the antibody hinges are as follows:

| | |
|---|---|
| IgG4 short hinge: | KYGPPC ... |
| IgG4 long hinge: | VDKRVES ... |
| IgG2: | ERKCCV ... |

Primers incorporated restriction sites to enable later ligation into a high expression vector pDR2 ΔEF1α. (BamHl at the 5' end and EcoRV at the 3' end). DNA encoding the signal peptide and either the first three or four SCR domains of hDAF was amplified by PCR using plasmid containing DAF sequences as a template. The carboxy-terminal sequences of the DAF domains are as follows:

| | |
|---|---|
| 3SCR form: | ... PECREIY |
| 4SCR form (with IgG4 long hinge): | ... KSLTSK |
| 4SCR form (with IgG4 short hinge and IgG2): | ... PPPECRG |

Amplified DNA was separated from the template by electrophoresis on an agarose gel. The insert was extracted from the gel, cut with suitable restriction enzymes at sites encoded on the PCR primers (BamH1 at the 3' end and Xbal at the 5' end) and ligated into pDR2 ΔEF1α upstream of and in frame with DNA encoding the hinge and Fc domains of the human immunoglobulin. DNA proof-reading polymerase was used in the PCR reactions and sequencing confirmed that no errors had been introduced by PCR. CHO cells were transfected using lipofectamine (Life Technologies) according to the manufacturer's instructions and stable lines were established by selection with 400µg/ml Hygromycin B (Life Technologies). Supernatant was collected and passed over a Prosep A column (Bioprocessing Ltd, Consett, UK) to purify the fusion protein. The column was washed with PBS and with 0.1M citrate buffer pH5.0 to remove contaminating bovine Ig and the fusion protein was eluted with 0.1M Glycine/HCl pH2.5. Eluted protein was neutralised with Tris, concentrated by ultrafiltration and dialysed into PBS. The purified proteins were analysed by SDS PAGE.

### Functional Assay Protocols

### Protocol Example 1: - Functional Analysis of DAF-Ig and sDAF

In order to assess function of rat DAF, antibody coated sheep erythrocytes (E; 2% (v:v)) were prepared by incubating cells in PBS for 30 minutes with 1/500 dilution of rabbit anti-sheep E (Amboceptor, Behring Diagnostics GmbH). Sensitised E were washed three times in GVB (Gelatin Veronal Buffer comprising CFD [C-fixation Diluent with added 0.1% (w/v) gelatin (Immunol 100 463 (2000)]) and re-suspended to 2%. In order to determine a concentration of rat serum giving partial lysis (50-80%), antibody coated sheep E (EA) were incubated for 30 minutes at 37°C with different dilutions of serum. Following pelleting of cells by centrifugation, amount of lysis was quantitated by adding an aliquot of supernatant (50 µl) to water (100 µl) and measuring absorbance at 415nm. Control samples were prepared by adding buffer only (0% control) or 0.1% Triton X100 (100% control) to the E instead of serum. % lysis was calculated as follows: % lysis = 100x(A415 sample-A415 0% control)/(A415 100% control-A415 0% control). To test function of the recombinant inhibitors, EA were incubated with the predetermined dilution of rat serum giving 50-80% lysis and different dilutions of the test protein. Following incubation at 37°C, % lysis was determined as described above.

### Protocol Example 2: - Functional Analysis of CD59

Guinea pig E(GPE) were washed and re-suspended in GVB at 2% (v:v). These were incubated for 30 minutes at 37°C with an equal volume of 25% (v:v) normal human serum from which C8 had been depleted be passage over a monoclonal anti-C8 affinity column. The resulting cells (GPE-C5b7) were washed and re-suspended at 2% in PBS.10mM EDTA. The amount of rat serum giving 50-80% lysis was determined by incubating GPE-C5b7 for 30 minutes at 37°C with dilutions of rat serum in PBS/EDTA. In order to assess function of soluble CD59, GPE-C5b7 were incubated in PBS, EDTA with dilutions of the test reagent and the predetermined concentration of rat serum. 0% and 100% controls were included and %lysis was determined as described above.

### Illustrative Results of Known Studies

### Illustrative Example 1: - In vivo clearance of DAF-Ig is slowed compared to sDAF

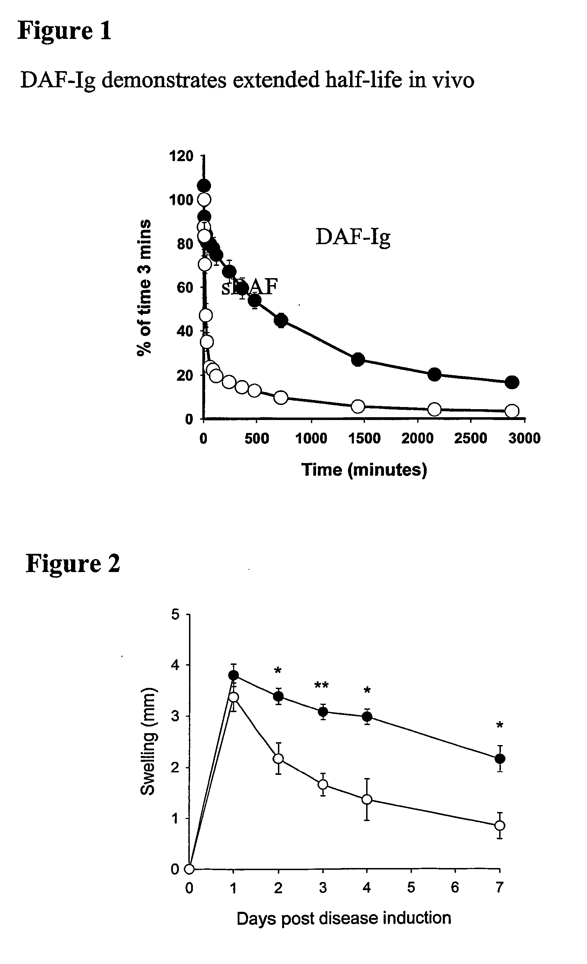

In order to study the effect of the Fc domain (immunoglobulin crystallisable fragment) on clearance of soluble DAF (sDAF), DAF-Ig and sDAF were radiolabelled with ¹²⁵I. Animals were administered with a single dose of either reagent and samples of blood were removed at certain timepoints. Protein was precipitated using TCA and protein bound counts were measured in a gamma counter. At 1 hour following administration, sDAF levels were down to 20% of that seen at 3 minutes, DAF-Ig levels were still 80% of those at 3 minutes, demonstrating the enhancement of half life as a consequence of fusion to Ig domains **(****Figure 1****).**

### Illustrative Example 2: - DAF-Ig delays onset and inhibits progression in antigen ⁱnduced arthritis (AIA)

AIA was induced in Wistar rats (Goodfellow et al, Clinical and Experimental Immunology (1997), 110, 45). Briefly, methylated BSA (mBSA) was introduced into the right knee of five rats pre-immunised with mBSA; 0.45mg DAF-Ig or the same volume of saline (control animals) was included with the antigen. Disease progression was monitored by comparing swelling of the right knee to that of the left, over the course of a week. Rat DAF-Ig caused a significant reduction in swelling and disease severity compared to control animals from day 2 onwards **(****Figure 2****).** These results indicate the long-term effects of DAF-Ig fusion proteins in vitro.

### Results of New Studies Relating to the Invention

### Example 1:- In vitro functional analyses of DAF-Ig, sDAF, CD59-Ig and CD59 - Cleavage by Papain

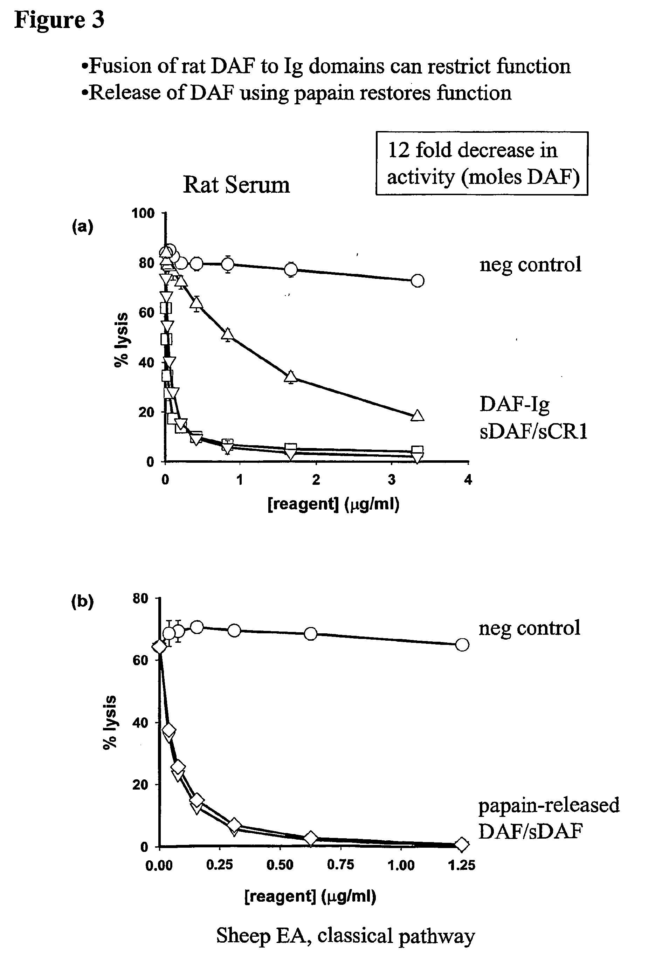

The ability of DAF-Ig and sDAF to inhibit the classical pathway of C was analysed using a haemolysis assay and was compared to inhibition of lysis achieved with sCR1 . Both sDAF and sCR1 were powerful inhibitors of lysis, while DAF-Ig showed a reduced ability to inhibit lysis **(****Figure 3****).** Tests using papain to cleave Ig from DAF indicated that the functional activity of DAF in vitro could be restored by removal from the Ig. This was shown by the fact that DAF released from Ig domains by digestion with papain, had identical activity to sDAF secreted from CHO cells.

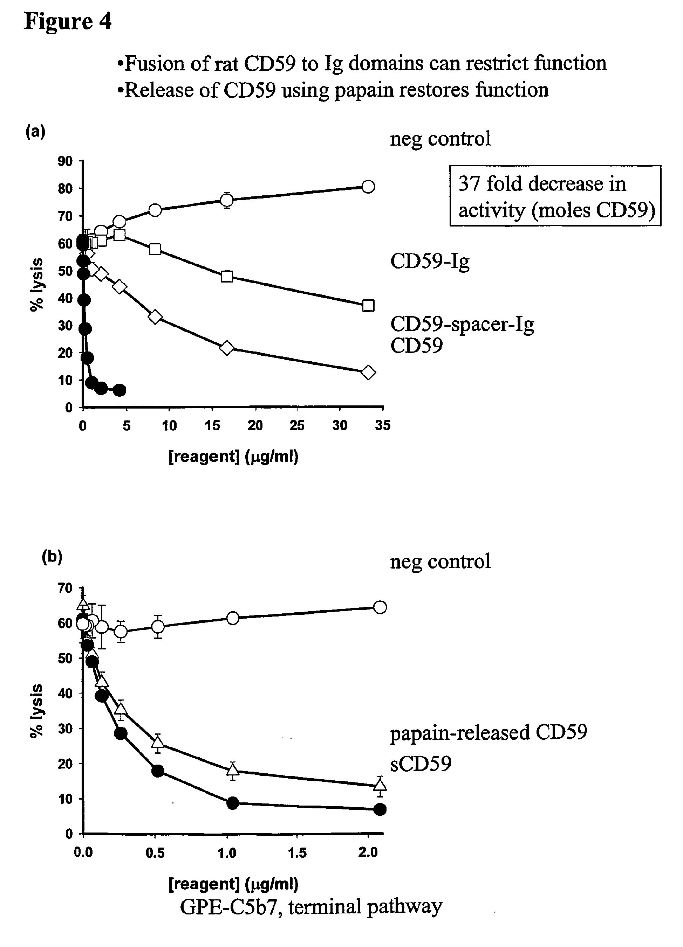

The ability of CD59-Ig and CD59-spacer-Ig to inhibit C was also tested using haemolysis assays specific for the terminal pathway. Again, the fusion protein showed a much lowered ability to inhibit MAC formation when compared to CD59 released from CD59-Ig using papain. This, like the DAF analysis, indicated that cleavage of the Ig from the CD59 increased activity **(****Figure 4****).** Further, the presence of the spacer domain indicated its ability to modify the activity of the regulatory moiety, and implicated steric factors in loss of regulatory function in the prodrug. The presence of a spacer domain enhanced regulatory function of CD59-Ig although activity was still low compared to sCD59.

**Figures 3** **and** **4** show that both DAF-Ig and CD59-Ig had less complement regulatory capacity than the soluble forms lacking the Fc. It is likely that this is due to steric constraints in which the active site of the regulatory proteins cannot access and bind the large multimolecular substrate, be it the C3/C5 convertase or MAC. This is supported by the observation that enzymatic removal of the Fc domains restores full function to the released regulatory protein.

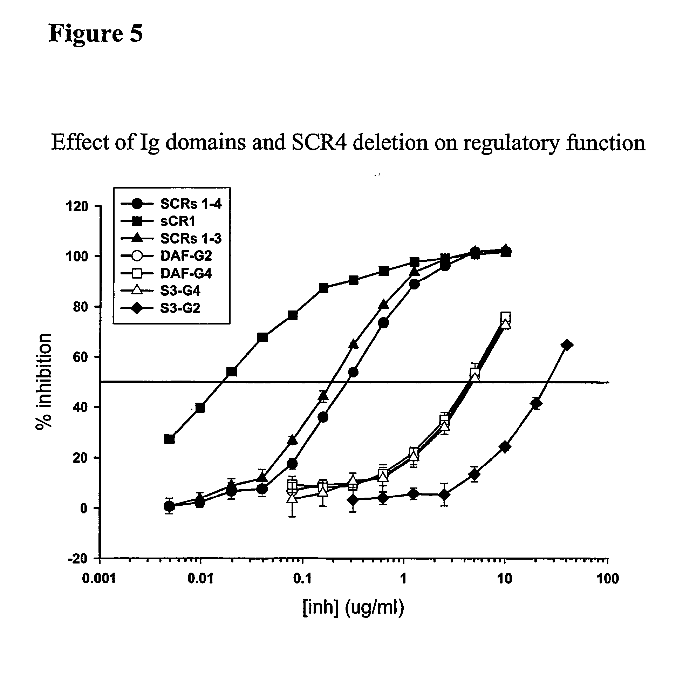

In addition to modification of activity of a therapeutic reagent using a spacer domain as described above and shown on **Figure 4****,** the type of antibody used as the carrier protein can influence the effect of a therapeutic reagent. This is presumably due to the steric influence of variations in the hinge region of an antibody. Studies as shown in **Figure 5****,** indicate that Ig with less flexible hinge regions, for example IgG2, cause more restriction on the functional activity of a linked therapeutic reagent *in vitro.* Deletion of the fourth SCR of human DAF further restricted functional activity of the CReg **(****Figure 5****).**

### Example 2: Preparation of New Fusion Proteins incorporating IGD1, IGD2 and IGD75 Cleavage Sites

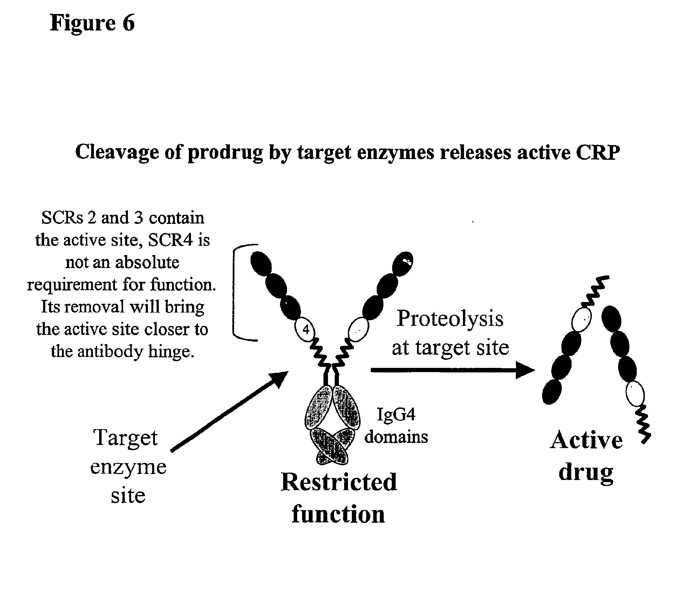

**Figure 6** shows a diagrammatic representation of a prodrug form of a therapeutic reagent according to the invention, showing the position of targeted enzyme cleavage sites between a regulatory moiety and the hinge region of a carrier protein.

In this Example, studies were made using cleavage sites appropriate to enzymes involved in inflammatory disease such as arthritis. MMPs and aggrecanases are involved in inflammation of the joints and they destroy cartilage by proteolysis of the major constituent proteoglycan, aggrecan. Polypeptides containing three different cleavage sites for some of these enzymes (MMP3, MMP8 and aggrecanase (ADAM-TS4)) were incorporated into CReg-Ig fusion proteins between the CReg and the hinge.

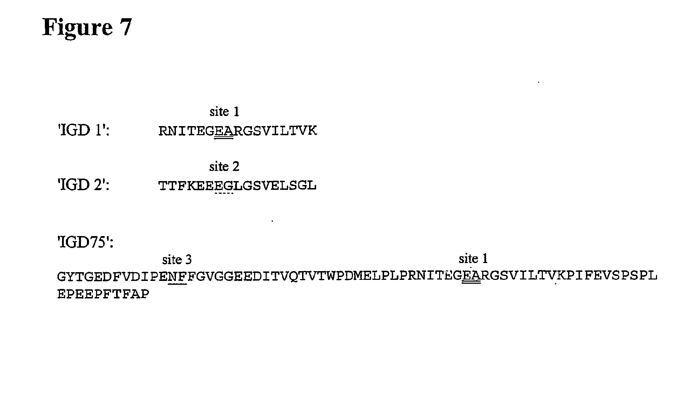

The length of the polypeptide was restricted to 17 amino acids each (termed here IGD1 and IGD2, each incorporating a different cleavage site) or 75 amino acids (termed here IGD75, incorporating two cleavage sites: the site from IGD1 and another site). Scrambled IGD is a control sequence containing no cleavage sites. **(Sequences shown in** **Figure 7****).**

In the case of IGD1, the amino acid sequence is RNITEGEARGSVILTVK; IGD2 has the amino acid sequence TTFKEEEGLGSVELSGL; and IGD75 has the amino acid sequence: GYTGEDFVDIPENFFGVGGEE-DITVQTVTWPDMELPLPRNITEGEARGSVILTVKPIFEVSPSPLEPEEPFTFAP.

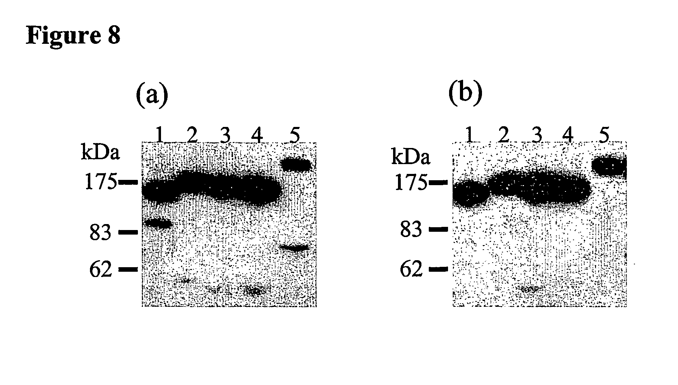

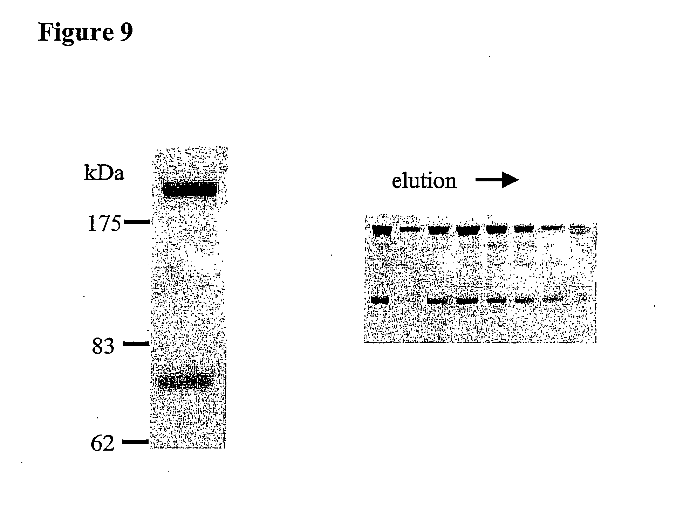

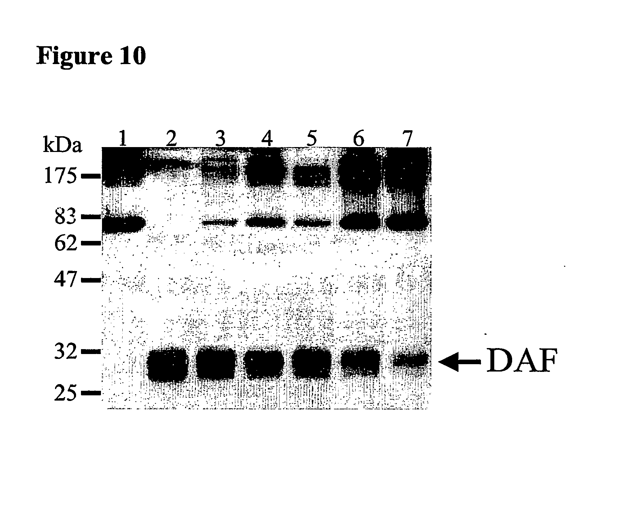

To incorporate the enzyme sites, complementary DNA oligomers encoding the short IGD sites with suitable restriction sites at both ends were used (BamH1). These were annealed together, restricted with BamH1 and ligated into the expression vector between DNA encoding DAF and the antibody hinge. The longer stretch of DNA encoding 75 amino acids of IGD was amplified using PCR from a plasmid template and similarly ligated into the vector at the BamH1 site (primers incorporated the restriction site). CHO cells were transfected as described above and the culture supernatant was collected. **Figure 8** shows Western Blot analysis using anti-human Ig (goat anti-human Fc - HRPO conjugated; 1 : 1000 dilution, available from Sigma) to demonstrate the presence of DAF-Ig in culture supernatant which contained target enzyme cleavage sites. In the case of aggrecan, several portions of the IGD can be included. **Figure 9** shows a SDS-PAGE analysis of the purified DAF-Ig prodrug. **Figure 10** shows digestion of the DAF-Ig prodrug with MMP3 and MMP8 enzymes and detection of released DAF in a western blot.

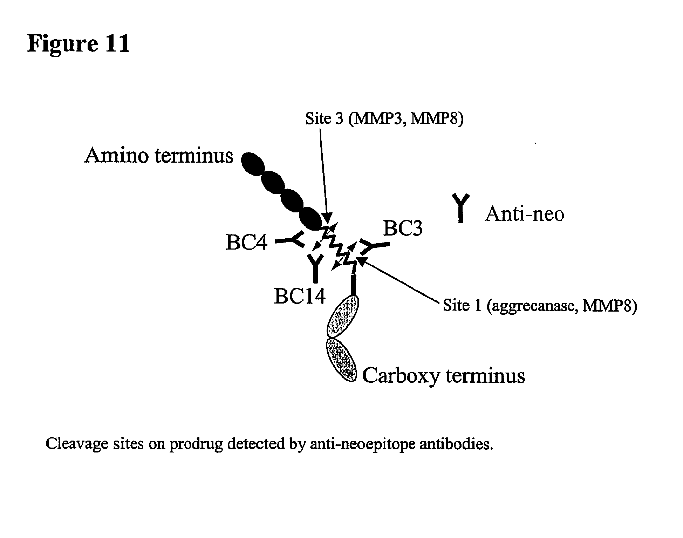

A schematic showing various cleavage sites of a therapeutic reagent according to the invention and their detectability by anti-neo-epitope antibodies is shown in **(****Figure 11****).**

Secreted fusion proteins were purified by Protein A affinity chromatography. The fusion protein containing IGD 75 was further purified by gel filtration on a Superose 12 gel filtration column (Amersham-Pharacia Biotech AB), equilibriated with 50Mm Tris pH 7.5, 100mM NaCl, 10mM CaCl_{2.}2H₂O **(****Figure 9****).** The eluted protein was digested with MMP3, MMP8 and aggrecanase **(****Figure 10****).**

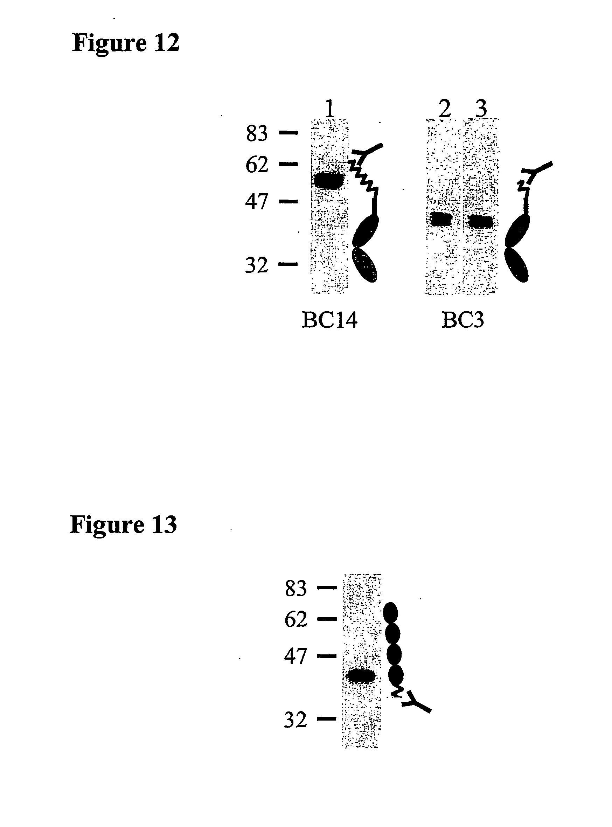

The therapeutic reagent (3.5µg) was incubated at 37°C for 24 hours with 0.3µg neutrophil MMP8 (Calbiochem) or with recombinant aggrecanase. The sample was lyophilised, re-dissolved in reducing SDS PAGE loading buffer and subjected to SDS PAGE and Western blot (1µg protein/lane). The blots were probed with anti-neoepitope antibodies that recognise the 'cut-ends' of aggrecan. Primary antibodies were detected with HRPO-linked secondary antibodies and bands were visualised using enhanced chemiluminescence (ECL). The antibody BC3 (Hughes et al Biochem J 305 700 (1995)) recognises the new N-terminus formed following cleavage at the major aggrecanase cleavage site (site 1 in **Figure 11****);** the antibody BC14 (Caterson et al in Acta Orthop Scand Suppl 266 121 (1995)) recognises the new N-terminus formed following cleavage at the major MMP site (FFG - site 3 in **Figure 11****).** BC13 recognises the new C-terminus following cleavage at the major aggrecanase site (EGE). BC4 recognises the new C-terminus created following cleavage at the major MMP site (PEN - site 3 in Figure 7). The antibodies were used to probe the blots at 1 : 100 (tissue culture supernatant). Cleavage of the prodrug by MMP8 at both enzymes sites was detected **(****Figure 12****,** lanes 1 and 2) and also cleavage by aggrecanase at the aggrecanase site **(****Figure 12****,** lane 3).

The therapeutic reagent in prodrug form was digested with MMP8 and analysed by Western blot as described above. The blot was probed with an anti-neo-epitope antibody that recognises the new C-terminus formed following cleavage at the major MMP site, BC4 recognises the new C-terminus formed following cleavage at the major MMP site (PEN - site 3); this protein fragment comprises hDAF and a small stretch of aggrecan IGD **(****Figure 13****).**

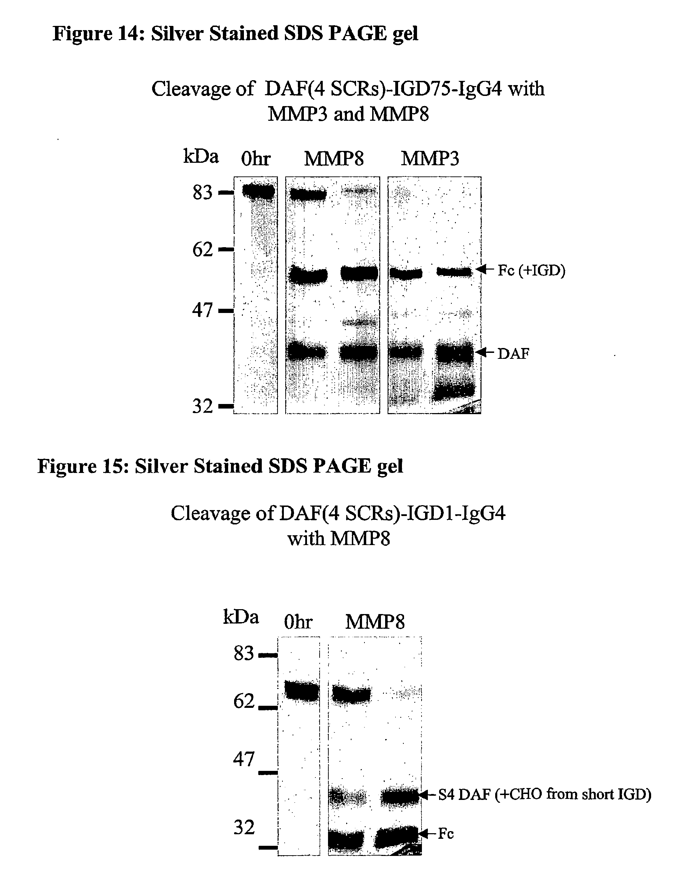

The results shown in **Figures 11 to 13** demonstrate that short enzyme sites can be incorporated into Ig-fusion proteins and that the active agent can be released following cleavage by the target enzyme. **Figure 14** shows a silver-stained SDS-PAGE analysis of the cleavage by MMP8 and MMP3 of the prodrug comprising DAF attached to the IgG4 Fc via the IGD75 linker (Figure 7). **Figure 15** shows a silver-stained SDS-PAGE analysis of the cleavage by MMP8 of the prodrug comprising DAF attached to the IgG4 Fc via the IgD 1 linker (Figure 7).

### Example 3: Generation of S3-DAF-IgG2 prodrug containing 'DIPEN' enzyme site

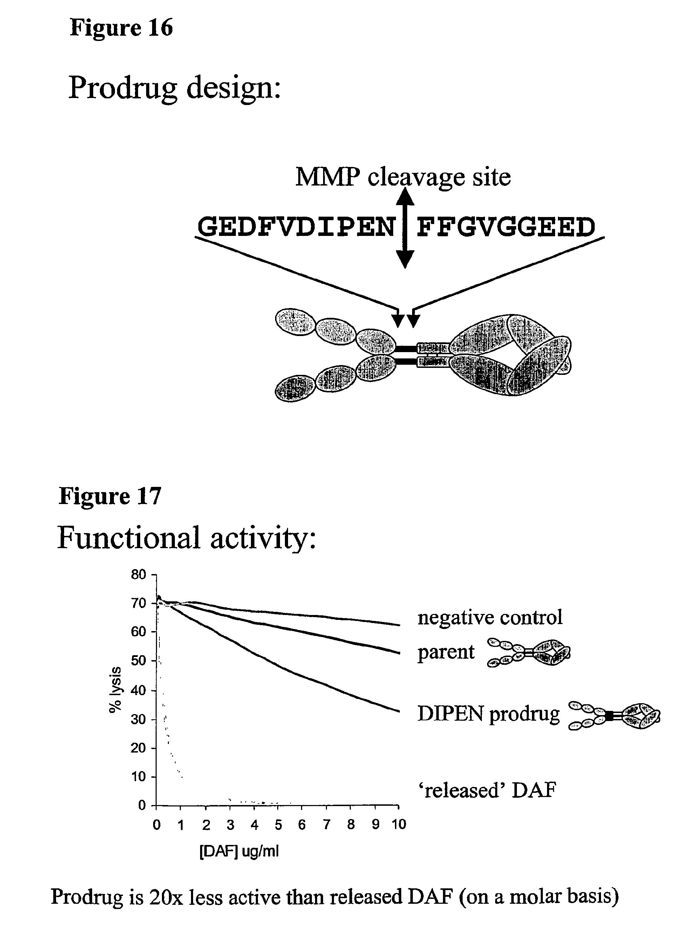

DNA encoding the hinge (starting amino acids ERKCCV...), CH2 and CH3 domains of human IgG2 was amplified by RT-PCR from peripheral blood mononuclear cell total RNA and ligated into the high expression vector PDR2 Δ EF1α (Charreau et al in Transplantation 58 1222 (1994). DNA encoding the signal peptide and first three SCR of human DAF (finishing amino acids ...CREIY) was amplified by PCR from plasmid template and ligated into the vector upstream of and in frame with DNA encoding the antibody hinge, as described in Method Example 5. CHO cells were transfected and fusion protein (S3 DAF-IgG2; also termed 'parent' molecule, not having cleavage site) was purified as described in Method Example 1. A prodrug form (termed the 'DIPEN prodrug') of the reagent was prepared as described in Example 2 using BamH1 restriction sites by inserting DNA (purchased oligomers) encoding the enzyme site here termed 'DIPEN' between DNA encoding DAF and the Ig. The sequence of the inserted enzyme site is GEDFVDIPENFFGVGGEED; this is illustrated in **Figure 16** where the cleavage site is indicated (immediately upstream of the DIPEN sequence). This site is cleaved by most MMPs.

### Functional activity of S3 DAF-IgG2 and corresponding DIPEN prodrug

S3 DAF-IgG2 (parent) and the DIPEN prodrug were purified by protein A affinity chromatography and gel filtration on a Superose 12 column as described in Example 2. Functional activity was assessed by inhibition of lysis of antibody coated sheep erythrocytes (EA) essentially as described in Protocol Example 1, ensuring that the buffer composition (GVB, PBS or Tris/Ca²⁺) was equivalent in each incubation **(****Figure 17****).** Control incubations included a non-regulatory SCR-containing fusion protein (negative control) and a three SCR form of DAF produced in yeast. Calculation of IH₅₀ and adjustment for molarity (equivalent moles of DAF) indicated that the DIPEN prodrug was approximately 20 fold less active than the three SCR form of DAF. Incorporation of the enzyme site acted as a 'spacer' domain and restored some activity to S3 DAF-IgG2 (compare 'parent' to DIPEN prodrug).

### Stability of S3 DAF-IgG2 and DIPEN prodrug

Both reagents were gel filtered into Tris/NaCl/Ca²⁺ as described in Example 2 **(****Figure 18****,** filtration of prodrug), and incubated for 24 hours at 37°C. S3 DAF-IgG2 was incubated in the presence of MMP3 (Calbiochem, recombinant catalytic domain) to assess non-specific cleavage by the target enzyme. Aliquots were removed from the incubations at the specified time-points and stored frozen in reducing SDS PAGE loading buffer until the end of the experiment. Samples were run on a 10% gel and silver stained according to the method of Morrissey in Anal Biochem 117 307-10 (1980) **(****Figure 19****).** Both molecules were stable stored at 37°C, the parent molecule was also stable in the presence of MP3.

### Cleavage of DIPEN prodrug with MMP3

The prodrug was incubated for 1, 2.5, 5, 7.5 and 24 hours with MMP3 at the concentrations specified in the following table.

| **Prodrug (µg/ml)** | **MMP3 (µg/ml)** | **Ratio Prodrug : MMP3** |
|---|---|---|
| | | |
| 100 | 10 | 10 : 1 |
| 100 | 2 | 50 : 1 |
| 200 | 2 | 100 : 1 |

Aliquots of each incubation were analysed by silver staining as described above. The prodrug was cleaved by MMP3 even at ratios of 100:1 (w:w) **(****Figure 20****).** The upper cleavage band (≈ 35kDa) represents the released Fc domains (confirmed by Western blot); the lower cleavage product (≈ 30kDa) is the released DAF (three SCRs). The DIPEN prodrug was also cleaved by MMP8 (not shown) and could therefore form a target for a multitude of metalloproteases.

### Western blot detection of released DAF and neo-epitope formation

In order to confirm that the prodrug had been cleaved at the metalloprotease site, portions of the incubations **described** above **(****Figure 20****)** were run on an 11% reducing SDS PAGE gel, Western blotted and probed with BC14 to detect the neo-epitope (antibody 'side') formed following cleavage (as described in Example 2 and **Figure 11****).** BC14-reactive neo-epitope was detected following incubation of DIPEN prodrug with MMP3, but not when the prodrug was incubated alone (prodrug control) or when the parent molecule was incubated with MMP3 **(****Figure 21****).** Samples from a similar incubation (DIPEN prodrug at 200µg/ml, MMP3 at 5µg/ml) were analysed by non-reducing SDS PAGE and Western blot using a monoclonal anti-DAF antibody. The prodrug was incubated in the absence of MMP3 as a control. Released DAF was detected when the prodrug was incubated with enzyme **(****Figure 22****).**

### Restoration of function following cleavage

In order to demonstrate that incubation of the DIPEN prodrug with MMP3 restored complement-regulatory activty, the following incubations were set up:
(1) Prodrug (200µg/ml)
(2) Prodrug (200µg/ml); MMP3 (5µg/ml)
(3) BSA (200µg/ml); MMP3 (5µg/ml)

Proteins were incubated for up to 24 hours and analysed by SDS PAGE and silver stain **(inset to** **Figure 23****).** 3 hour and 6 hour incubations of prodrug (with and without MMP3) and 6 hour incubation of BSA (with MMP3) were analysed by haemolysis assay as described in Protocol Example for complement-regulatory activity. Per cent lysis and inhibition (compared to negative control: non-regulatory fusion protein) were calculated **(****Figure 23****).** MMP3 in the BSA incubation had no effect on complement-mediated lysis of EA. As can be seen in **Figure 23****,** incubation of the DIPEN prodrug with MAP3 for 3 hours restored almost all DAF activity.

### Cleavage of anti-complement prodrug using native enzyme release from activated chondrocytes

In order to determine whether enzyme released from chondrocytes treated with pro-inflammatory cytokines, rather than purified enzyme, could cleave the prodrug, experiments were carried out using the therapeutic reagent described in **Example 2** (comprising 4 SCRs of human DAF fused to human IgG4 and'IGD75' as the inserted enzyme site **(****Figure 7****)).**

By a method analogous to that described by Hughes et al in J Biol Chem 272 20269 (1997)), pig chondrocytes were embedded in agarose and cultured in the presence of various cytokines (retinoic acid, EL-1, TNF-α) and prodrug. After 4 days, media samples were dialysed against water, lyophilised to dryness and reconstituted with reducing SDS- PAGE loading buffer containing 10% (v/v) mercaptoethanol. Samples were separated on 10% SDS-PAGE gels, transferred to nitrocellulose membranes and Western blot analysis was performed with anti-neo-epitope antibodies. BC3 detects cleavage at the aggrecanase site, whereas BC4 (Hughes et al Biochem J 305 700 (1995)) and BC14 detect cleavage at the MMP site (Figure 11).

**Figures 24A** illustrate cleavage of the prodrug by aggrecanase released from cultures stimulated with either retinoic acid, IL-1α or TNF using 3.4 µg of prodrug and anti-ARG (BC3) monoclonal (1:100). Cleavage of the prodrug at the aggrecanase site was only evident in the presence of stimulatory cytokines.

In addition, **Figure 24B** illustrates an IL-1 dose-dependent cleavage by aggrecanase of prodrug, detected using BC3. **Figure 24C** illustrates cleavage at the MMP site using BC14; cleavage was evident in the presence of stimulating cytokines.

### Conclusion

These data illustrates the preparation of a new prodrug, ideally based on an IgG2 backbone, and containing a short cleavage site for metalloproteases. Whilst insertion of the enzyme site into the 'parent' molecule restored some function, the prodrug showed a marked reduction in activity compared to released DAF. This enzyme site could be further truncated to retain as much inhibition of function as possible in the prodrug; the parent molecule (having no enzyme site) showed almost two logs reduction in function. The DIPEN prodrug was susceptible to cleavage by several metalloproteases tested; release of DAF and formation ofneo-epitopes following digestion was demonstrated by silver stain and Western blot. The cleavage reaction was almost complete as assessed by silver stain. Incubation of the prodrug with MMP3 restored almost complete complement-regulatory function to the reagent. Importantly, analysis of the cleavage of these reagents using native enzyme released from target cells, chondrocytes is given.

Using a culture system, cleavage of DAF-IGD75-IgG4 at both the metalloprotease and aggrecanase site has been demonstrated. Cleavage was triggered using various pro-inflammatory cytokines.

## Claims

1. A therapeutic reagent to control one or more reactions of the immune system in a host, said therapeutic reagent comprising:
i. at least one regulatory moiety that is a complement regulatory protein (C Reg) or a functional fragment thereof;
ii. a carrier protein which is an antibody, or a fragment thereof, and which renders said C Reg inactive or substantially inactive; and
iii. positioned therebetween at least one enzyme cleavage site for an enzyme present at sites of inflammation or immune pathology;
whereby
when the regulatory moiety is at, or adjacent, a target organ or tissue in the host, said cleavage site is cleaved, freeing the regulatory moiety from the carrier protein and restoring its regulatory activity.

2. A therapeutic reagent according to claim 1 wherein:
said cleavage site is a substrate for a matrix metalloproteinase (MMP) or an aggrecanase; whereby
at sites where MMP's or aggrecanase are active in the host said cleavage site is cleaved and said regulatory moiety is freed from the carrier protein and so able to perform its therapeutic function.

3. A therapeutic reagent according to claim 1 wherein:
said cleavage site comprises a substrate for at least one enzyme of the Complement system; whereby
when said therapeutic reagent is at or adjacent a site in the host where Complement is active said cleavage site is cleaved so freeing the regulatory moiety from the carrier protein and enabling it to perform its regulatory activity.

4. A therapeutic reagent according to claims 1-3, wherein the C Reg acts either as:
a decay accelerating factor, or a cofactor for the plasma protease factor I, or to inhibit formation of membrane attack complex, or a combination therof.

5. A therapeutic reagent according to any preceding claim, wherein said reagent has more than one regulatory moiety, two of which have different activities.

6. A therapeutic reagent according to any preceding claim, wherein said cleavage site comprises a plurality of cleavage sites.

7. A therapeutic reagent according to claims 2, 4, 5 or 6, wherein said cleavage site comprises a substrate for MMP3 or MMP8.

8. A therapeutic reagent according to any preceding claim, wherein at least one of said cleavage sites comprises a part of the inter-globular-domain (IGD) of aggrecan.

9. A therapeutic reagent according to claim 8, wherein said cleavage site comprises 17-75 amino acids of said IGD.

10. A therapeutic reagent according to claim 8, wherein the cleavage site comprises the minimal aggrecanase cleavage site in said aggrecan IGD (inter globular domain).

11. A therapeutic reagent according to claims 2, 4, 5 or 6, wherein the cleavage site includes the amino acid sequence DIPEN.

12. A therapeutic reagent according to claims 2, 4, 5, 6 or 11, wherein the cleavage site includes the amino acid sequence GEDFVDIPENFFGVGGEED.

13. A therapeutic reagent according to claims 2, 4, 5 or 6, wherein the cleavage site includes the amino acid sequence RNITEGEARGSVILTVK.

14. A therapeutic reagent according claims 2, 4, 5 or 6, wherein the cleavage site includes the amino acid sequence TTFKEEGLGSVELSGL.

15. A therapeutic reagent according to claims 2, 4, 5 or 6, wherein the cleavage site includes the amino acid sequence

GYTGEDFVDIPENFFGVGGEEDITVQTVTWPDMELPLPRNITEGEARGSVILTVK

PIFEVSPSPLEPEEPFTFAP.

16. A therapeutic reagent according to any preceding claim, wherein said antibody is human immunoglobulin IgG4, IgG2 or IgG1.

17. A therapeutic reagent according to claim 16 wherein one or more Fab arms of said immunoglobulin contains, or is replaced by, a further regulatory moiety.

18. A therapeutic reagent according to claims 16 or 17, wherein one or more arms of the Fab of the immunoglobulin contains or is replaced by a targeting moiety.

19. A therapeutic reagent according to claim 18, wherein the targeting moiety comprises one or more membrane targeting molecules.

20. A therapeutic reagent according to claim 19, wherein the targeting moiety comprises one or more addressins that is incorporated between the regulatory moiety and the cleavage site so that, following cleavage, said addressin directs the regulatory moiety to its target site.

21. A therapeutic reagent according to claim 20, wherein the addressin is APT542.

22. A therapeutic reagent according to any preceding claim, wherein the cleavage site is positioned between the regulatory moiety and a hinge region of the carrier protein.

23. A method of making a therapeutic reagent according to any preceding claim, comprising expressing protein from cells transformed or transfected with at least one nucleic acid molecule encoding said therapeutic reagent.

24. Use of a therapeutic reagent according to Claims 1 - 22, in the preparation of a medicament for the treatment of disease.

25. Use according to Claim 24 wherein the disease to be treated includes one or more of: inflammatory, immunological, traumatic, ischaemic diseases or other disorders in which Complement contributes to pathology.

26. Use according to claim 25 wherein the disease is one or more of: rheumatoid arthritis, systemic lupus erythematosis, glomerulonephritis, multiple sclerosis, adult respiratory distress syndrome (ARDS), ischemia-reperfusion injury, demyelination, myaesthenia gravis, Arthus reaction or rejection in transplantation

27. A pharmaceutical composition including a therapeutic reagent according to claims 1-22, which is combined with a pharmaceutically acceptable carrier.

## Patentansprüche

1. Therapeutisches Reagenz zur Kontrolle einer oder mehrerer Reaktion(en) des Immunsystems in einem Wirt, wobei das therapeutische Reagenz umfasst:
i. mindestens eine regulatorische Einheit, bei der es sich um ein Komplement-regulatorisches Protein (C Reg) oder ein funktionelles Fragment davon handelt,
ii. ein Trägerprotein, bei dem es sich um einen Antikörper handelt, oder ein Fragment davon, und welches das C Reg inaktiv oder im Wesentlichen inaktiv macht, und
iii. positioniert dazwischen mindestens eine Enzymspaltstelle für ein Enzym, das an Orten einer Entzündung oder einer Immunpathologie vorliegt, wodurch,
wenn sich die regulatorische Einheit bei einem Zielorgan oder -gewebe in dem Wirt oder angrenzend daran befindet, die Spaltstelle gespalten wird, wobei die regulatorische Einheit von dem Trägerprotein freigesetzt wird und deren regulatorische Aktivität wiederhergestellt wird.

2. Therapeutisches Reagenz nach Anspruch 1, bei dem:
die Spaltstelle ein Substrat für eine Matrix-Metalloproteinase (MMP) oder eine Aggrecanase ist, wodurch
an Stellen, an denen MMP's oder Aggrecanase in dem Wirt aktiv sind/ist, die Spaltstelle gespalten wird und die regulatorische Einheit von dem Trägerprotein freigesetzt wird und so deren therapeutische Funktion ausüben kann.

3. Therapeutisches Reagenz nach Anspruch 1, bei dem:
die Spaltstelle ein Substrat für mindestens ein Enzym des Komplementsystems umfasst, wodurch,
wenn sich das therapeutische Reagenz an einer Stelle in dem Wirt, an der das Komplement aktiv ist, oder angrenzend daran befindet, die Spaltstelle gespalten wird, so dass die regulatorische Einheit von dem Trägerprotein freigesetzt wird und deren regulatorische Aktivität ausüben kann.

4. Therapeutisches Reagenz nach einem der Ansprüche 1 bis 3, bei dem das C Reg entweder als ein zerfallsbeschleunigender Faktor oder als Cofaktor für den Plasmaproteasefaktor 1 wirkt oder die Bildung eines Membranangriffskomplexes hemmt, oder als eine Kombination davon wirkt.

5. Therapeutisches Reagenz nach einem der vorstehenden Ansprüche, wobei das Reagenz mehr als eine regulatorische Einheit aufweist, wobei zwei davon unterschiedliche Aktivitäten aufweisen.

6. Therapeutisches Reagenz nach einem der vorstehenden Ansprüche, bei dem die Spaltstelle eine Mehrzahl von Spaltstellen umfasst.

7. Therapeutisches Reagenz nach Anspruch 2, 4, 5 oder 6, bei dem die Spaltstelle ein Substrat für MMP3 oder MMP8 umfasst.

8. Therapeutisches Reagenz nach einem der vorstehenden Ansprüche, bei dem mindestens eine der Spaltstellen einen Teil der Interglobulardomäne (IGD) von Aggrecan umfasst.

9. Therapeutisches Reagenz nach Anspruch 8, bei dem die Spaltstelle 17 bis 75 Aminosäuren der IGD umfasst.

10. Therapeutisches Reagenz nach Anspruch 8, bei dem die Spaltstelle die minimale Aggrecanase-Spaltstelle in der Aggrecan-IGD (Interglobulardomäne) umfasst.

11. Therapeutisches Reagenz nach Anspruch 2, 4, 5 oder 6, bei dem die Spaltstelle die Aminosäuresequenz DIPEN umfasst.

12. Therapeutisches Reagenz nach Anspruch 2, 4, 5, 6 oder 11, bei dem die Spaltstelle die Aminosäuresequenz GEDFVDIPENFFGVGGEED umfasst.

13. Therapeutisches Reagenz nach Anspruch 2, 4, 5 oder 6, bei dem die Spaltstelle die Aminosäuresequenz RNITEGEARGSVILTVK umfasst.

14. Therapeutisches Reagenz nach Anspruch 2, 4, 5 oder 6, bei dem die Spaltstelle die Aminosäuresequenz TTFKEEGLGSVELSGL umfasst.

15. Therapeutisches Reagenz nach Anspruch 2, 4, 5 oder 6, bei dem die Spaltstelle die Aminosäuresequenz GYTGEDFVDIPENFFGVGGEEDITVQTVTWPDM-ELPLPRNITEGEARGSVILTVKPIFEVSPSPLEPEEPFTFAP umfasst.

16. Therapeutisches Reagenz nach einem der vorstehenden Ansprüche, bei dem der Antikörper menschliches Immunglobulin IgG4, IgG2 oder IgG1 ist.

17. Therapeutisches Reagenz nach Anspruch 16, bei dem ein oder mehrere Fab-Arm(e) des Immunglobulins eine weitere regulatorische Einheit enthält/enthalten oder durch eine solche ersetzt ist/sind.

18. Therapeutisches Reagenz nach Anspruch 16 oder 17, bei dem ein oder mehrere Arm(e) des Fab des Immunglobulins eine Targeting-Einheit enthält/enthalten oder durch eine solche ersetzt ist/sind.

19. Therapeutisches Reagenz nach Anspruch 18, bei dem die Targeting-Einheit ein oder mehrere Membran-Targeting-Molekül(e) umfasst.

20. Therapeutisches Reagenz nach Anspruch 19, bei dem die Targeting-Einheit ein oder mehrere Addressin(e) umfasst, das/die zwischen der regulatorischen Einheit und der Spaltstelle eingebaut ist/sind, so dass das Addressin nach der Spaltung die regulatorische Einheit zu deren Zielstelle führt.

21. Therapeutisches Reagenz nach Anspruch 20, bei dem das Addressin APT542 ist.

22. Therapeutisches Reagenz nach einem der vorstehenden Ansprüche, bei dem die Spaltstelle zwischen der regulatorischen Einheit und einer Gelenkregion des Trägerproteins positioniert ist.

23. Verfahren zur Herstellung eines therapeutischen Reagenzes nach einem der vorstehenden Ansprüche, umfassend das Exprimieren von Protein von Zellen, die mit mindestens einem Nukleinsäuremolekül, welches das therapeutische Reagenz kodiert, transformiert oder transfiziert worden sind.

24. Verwendung eines therapeutischen Reagenzes nach einem der Ansprüche 1 bis 22 bei der Herstellung eines Medikaments zur Behandlung einer Erkrankung.

25. Verwendung nach Anspruch 24, bei der die zu behandelnde Erkrankung eine oder mehrere von Entzündungsstörungen, immunologischen Störungen, traumatischen Störungen, ischämischen Störungen oder anderen Störungen ist, bei denen das Komplement zur Pathologie beiträgt.

26. Verwendung nach Anspruch 25, bei der die Erkrankung eine oder mehrere von rheumatoider Arthritis, systemischem Lupus erythematodes, Glomerulonephritis, Multiple Sklerose, Schocklunge (ARDS), Ischämie-Reperfusionsschädigung, Demyelinisation, Erb-Goldflam-Syndrom, Arthus-Reaktion oder Abstoßung bei Transplantation umfasst.

27. Pharmazeutische Zusammensetzung, die ein therapeutisches Reagenz nach einem der Ansprüche 1 bis 22 umfasst, das mit einem pharmazeutisch verträglichen Träger kombiniert ist.

## Revendications

1. Réactif thérapeutique pour contrôler une ou plusieurs réactions du système immunitaire dans un hôte, ledit réactif thérapeutique comprenant :
i. au moins une fraction régulatrice qui est une protéine régulatrice du complément (C Reg) ou un fragment fonctionnel de celle-ci ;
ii. une protéine support qui est un anticorps, ou un fragment de celui-ci, qui rend ledit C Reg inactif ou sensiblement inactif ; et
iii. positionné entre elles, au moins un site de clivage d'enzyme pour une enzyme présente au niveau des sites d'inflammation ou de pathologie immunitaire ; par lequel lorsque la fraction régulatrice est au niveau de, ou adjacente à un organe cible ou un tissu dans l'hôte, ledit site de clivage est clivé, libérant la fraction régulatrice à partir de la protéine de support et restaurant son activité régulatrice.

2. Réactif thérapeutique selon la revendication 1, dans lequel :
- ledit site de clivage est un substrat pour une métalloprotéinase de matrice (MMP) ou une aggrécanase ; ce par quoi
au niveau de sites ou les MMP ou l'aggrécanase sont actifs dans l'hôte, ledit site de clivage est clivé et ladite fraction régulatrice est libérée de la protéine de support et est ainsi capable d'effectuer sa fonction thérapeutique.

3. Réactif thérapeutique selon la revendication 1, dans lequel
Ledit site de clivage comprend un substrat pour au moins une enzyme du système du Complément, ce par quoi lorsque ledit réactif thérapeutique est au niveau de ou adjacent à un site dans l'hôte au niveau duquel le complément est actif, ledit site de clivage est clivé, libérant ainsi la fraction régulatrice de la protéine de support et lui permettant d'effectuer son activité régulatrice.

4. Réactif thérapeutique selon l'une des revendications 1 à 3, dans lequel le C Reg agit soif : comme facteur d' accélération du déclin ou, comme cofacteur pour le facteur 1 de la protéase de la protéase du plasma, ou pour inhiber la formation d'un complexe d'attaque de membrane, ou une combinaison de ceux-ci.

5. Réactif thérapeutique selon l'une quelconque des revendications précédentes, dans lequel ledit réactif a plus d'une fraction régulatrice, dont deux ont des activités différentes.

6. Réactif thérapeutique selon l'une quelconque des revendications précédentes, dans lequel ledit site de clivage comprend une pluralité de site de clivage.

7. Réactif thérapeutique selon l'une des revendications 2, 4, 5 ou 6, dans lequel ledit site de clivage comprend un substrat pour MMP3 ou MMP8.

8. Réactif thérapeutique selon l'une quelconque des revendications précédentes, dans lequel au moins l'un desdits sites de clivage comprend une partie du domaine interglobulaire (IGF) de l'aggrécane.

9. Réactif thérapeutique selon la revendication 8, dans lequel ledit site de clivage comprend 17-75 acides aminés dudit IGD.

10. Réactif thérapeutique selon la revendication 8, dans lequel le site de clivage comprend le site de clivage d'aggrécanase minimal dans ledit IGD d'aggrécane (domaine inter globulaire).

11. Réactif thérapeutique selon les revendications 2, 4, 5 ou 6, dans lequel le site de clivage comprend la séquence d'acides aminés DIPEN.

12. Réactif thérapeutique selon les revendications 2, 4, 5, 6 ou 11, dans lequel le site de clivage comprend la séquence d'acides aminés GEDFVDIPENFFGVGGEED.

13. Réactif thérapeutique selon les revendications 2, 4, 5 ou 6, dans lequel le site de clivage comprend la séquence d'acide aminé RNITEGEARGSVILTVK.

14. Réactif thérapeutique selon l'une des revendications 2, 4, 5 ou 6, dans lequel le site de clivage comprend la séquence d'acides aminés TTFKEEGLGSVELSGL.

15. Réactif thérapeutique selon l'une des revendications 2, 4, 5 ou 6, dans lequel le site de clivage comprend la séquence d'acides aminés :

GYTGEDFVDIPENFFGVGGEEDITVQTVTWPDMELPLPRNITEGEARGSVILTVKPIFE
VSPSPLEPEEPFTFAP.

16. Réactif thérapeutique selon l'une quelconque des revendications précédentes, dans lequel ledit anticorps est l'immunoglobuline humaine IgG4, IgG2 ou IgG1.

17. Réactif thérapeutique selon la revendication 16, dans lequel un ou plusieurs bras Fab de ladite immunoglobuline contient, ou est remplacé par, une autre fraction régulatrice.

18. Réactif thérapeutique selon les revendications 16 ou 17, dans lequel un ou plusieurs bras du Fab de l'immunoglobuline contient ou est remplacé par une fraction de ciblage.

19. Réactif thérapeutique selon la revendication 18, dans lequel la fraction de ciblage comprend une ou plusieurs molécules de ciblage de membrane.

20. Réactif thérapeutique selon la revendication 19, dans lequel la fraction de ciblage comprend une ou plusieurs addressines qui est incorporée entre la fraction régulatrice et le site de clivage de telle sorte qu'après le clivage, ladite addressine dirige la fraction régulatrice sur son site cible.

21. Réactif thérapeutique selon la revendication 20, dans lequel l'addressine est APT542.

22. Réactif thérapeutique selon l'une quelconque des revendications précédentes, dans lequel le site de clivage est positionné entre la fraction régulatrice et une région d'articulation de la protéine de support.

23. Procédé de fabrication d'un réactif thérapeutique selon l'une quelconque des revendications précédentes, comprenant l'expression de la protéine à partie de cellules transformées ou transfectées par au moins une molécule d'acide nucléique codant pour ledit réactif thérapeutique.

24. Utilisation des réactifs thérapeutiques selon les revendications 1-22, dans la préparation d'un médicament pour le traitement de maladies.

25. Utilisation selon la revendication 24, dans lequel la maladie devant être traitée comprend une ou plusieurs parmi : les maladies inflammatoires, immunologiques, traumatiques, ischémiques ou autre troubles dans lequel le Complément contribue à la pathologie.

26. Utilisation selon la revendication 25, dans lequel la maladie est une ou plusieurs parmi l'arthrite rhumatoïde, le lupus érythématosis systémique, la glomérulonéphrite, la sclérose en plaques, le syndrome de détresse respiratoire chez l'adulte (ARDS), la lésion d'ischémie-reperfusion, la démyélination, la miasthénie gravis, la réaction d'Arthus ou le rejet de transplantation.

27. Composition pharmaceutique comprenant un réactif thérapeutique selon les revendications 1-22 qui est combiné avec un support pharmaceutiquement acceptable.
